# FASCICULE DE BREVET EUROPEEN

(11) **EP 0 579 819 B1**
(45) Date de publication et mention de la délivrance du brevet: **21.12.2005**
(21) Numéro de dépôt: 93905369.0
(22) Date de dépôt: 05.02.1993
(51) Int. Cl.: C07D 211/86, C07D 401/06, C07D 401/04, C07D 401/12, C07D 401/10, A61K 31/44, C07D 309/30, C07D 309/32

(54) **NOUVEAU DERIVE DE LA PYRIDONE AYANT UNE AFFINITE POUR LE RECEPTEUR A L'ANGIOTENSINE II**
NEUER PYRIDON-DERIVAT DER EINE AFFINITÄT FÜR DEN ANGIOTENSIN-II-REZEPTOR HAT
NOVEL PYRIDONE DERIVATIVE HAVING AN AFFINITY FOR THE ANGIOTENSINE II RECEPTOR

(30) Priorité: 07.02.1992 FR 9201390
(43) Date de publication de la demande: 26.01.1994
(73) Titulaire: Aventis Pharma S.A., 92160 Antony (FR)
(72) Inventeur: FORTIN, Michel, F-75018 Paris (FR); HAESSLEIN, Jean-Luc, F-77181 Courtry (FR); HECKMANN, Bertrand, F-94230 Cachan (FR)
(86) Numéro de dépôt international: PCT/FR1993/000118
(87) Numéro de publication internationale: WO 1993/016049

(56) Documents cités:
- EP-A- 0 034 349
- EP-A- 0 040 082
- EP-A- 0 276 204
- EP-A- 0 324 377
- EP-A- 0 354 495
- EP-A- 0 403 159
- EP-A- 0 443 568
- EP-A- 0 445 811
- EP-A- 0 447 164
- EP-A- 0 465 368
- EP-A- 0 499 416
- WO-A-86/06374
- WO-A-91/00277
- WO-A-91/19697
- WO-A-92/00290
- US-A- 4 046 769
- US-A- 4 747 871
- US-A- 4 751 073
- US-A- 5 077 142

## Description

La présente invention concerne un nouveau dérivé de la pyridone, son application à titre de médicament et les compositions pharmaceutiques le renfermant.

La présente invention a pour objet le produit 2 - (hydroxyméthyl)-5-méthyl-3-phénylthio-1- [[2'- (1H-tétrazol-5-yl) (1,1'-biphényl)-4-yl] méthyl]-4- (1H) -pyridone, ainsi que les sels d'addition avec les acides minéraux et organiques ou avec les bases minérales et organiques dudit produit.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Les bases peuvent être des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclo-hexylamine, la morpholine, la benzylamine, la procaïne, la lysine, l'arginine, l'histidine, la N-méthylglucamine.

Le composé tel que défini ci-dessus ainsi que les sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Certains produits de la présente invention possèdent également des propriétés antagonistes pour le récepteur à l'endothéline et sont ainsi notamment antagonistes de l'effet vasoconstricteur de l'endothéline.

Les composés possèdent également la propriété d'améliorer les fonctions cognitives.

Ces propriétés justifient leur application en thérapeutique et l'invention a également pour objet à titre de médicament , le produit tel que défini ci-dessus, ainsi que les sels d'addition avec les acides minéraux ou organiques ou avec les bases minérales et organiques pharmaceutiquement acceptables dudit produit.

Les médicaments, objet de l'invention, peuvent être utilisés dans le traitement des affections cardiovasculaires présentant une altération de la vasomotricité : infarctus du myocarde, insuffisance cardiaque, insuffisance rénale, angine de poitrine, spasme vasculaire cérébral, maladie de Raynaud, hypertension artérielle et toutes les affections consécutives à une ischémie. Ces médicaments, objet de l'invention, pourraient également être utilisés pour le traitement du glaucome, de l'athérosclérose, de l'asthme et de différents types de spasmes viscéraux, ainsi qu'à titre de substances protectrices neuronales ou encore dans la prévention des resténoses post-angioplastie.

Ils peuvent également être utilisés dans le traitement de certains désordres gastro-intestinaux, gynécologiques et en particulier pour un effet relaxant au niveau de l'utérus.

Les médicaments, objet de l'invention peuvent également être utilisés dans le traitement des troubles de la mémoire et des fonctions cognitives, de l'anxiété, de la dépression, de la démence sénile et de la maladie d'Alzheimer.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les exemples suivants illustrent le procédé de préparation du produit de l'invention.

Le produit utilisé au départ de l'exemple 1 qui ne fait pas partie de l'invention a été préparé de la façon suivante :

### Préparation de l'exemple 1 : 4-acétoxy 1-benzyloxy 3-phénylthio 3-butène 2-one

### STADE A : 2-hydroxy 3-chloro 1-benzyloxypropane

On introduit :
- 15,7 ml de 1-chloro 2-3-époxypropane
- 41,4 ml d'alcool benzylique
- 0,2 ml de tétrachlorure d'étain
laisse revenir à la température ambiante puis chauffe jusqu'à une température d'environ 104°C pendant environ 3 heures.

On laisse revenir à la température ambiante, dilue avec du toluène, ajoute de l'eau, filtre, lave la phase organique avec du carbonate de sodium puis à nouveau avec de l'eau jusqu'à pH 7.

On sèche, filtre, élimine le toluène, distille et obtient 26,165 g de produit attendu (Eb 158°-159°C sous 16 ₘₘ/Hg)

| Analyses: | |
|---|---|
| Spectre IR (CHCl₃) | |
| - OH complexe | 3598 cm⁻¹ |
| aromatique | 1498 cm⁻¹ |

### STADE B : 1-benzyloxy 3-phénylthio 2-propanol

On introduit 25 ml de méthanol, ajoute 1,5 g de sodium, agite puis refroidit jusqu'à une température d'environ 0°C et ajoute:
- 7,3 ml de thiophénol
   puis
- 13 g du produit obtenu ci-dessus au stade A
   et
- 5 ml de méthanol.

On chauffe 12 heures au reflux à 68°C puis laisse revenir à température ambiante.

On dilue avec de l'eau, extrait par 3 fois avec de l'éther, lave la phase éthérée avec une solution aqueuse saturée en chlorure de sodium, sèche, filtre, élimine l'éther et obtient 18 g de produit attendu.

| Analyses | |
|---|---|
| Spectre IR (CHCl₃) | |
| - OH complexe | 3558 cm⁻¹ |
| phényl-C- | 1498 cm⁻¹ |
| phényl-S- | 1588 cm⁻¹ |

### STADE C : 1-benzyloxy 3-phénylthio 2-propanone

On introduit :
- 31,6 g du produit obtenu ci-dessus au stade B
- 215 ml d'anhydride acétique
   et
- 325 ml de diméthylsulfoxyde
et agite à température ambiante pendant 24 heures puis traite sous courant d'azote pour éliminer le sulfure de diméthyle.

On concentre, reprend avec de l'eau, extrait à l'éther, lave la phase organique avec une solution aqueuse saturée en chlorure de sodium, sèche, filtre, élimine l'éther.

Aprés chromatographie sur silice (hexane : 90/acétate d'éthyle : 10), on obtient 22,9 g de produit attendu.

| Analyses: | |
|---|---|
| Spectre IR (CHCl₃) | |
| C = O | 1725 cm⁻¹ |
| aromatiques | 1583 cm⁻¹ |
| | 1495 cm⁻¹ |
| | 1483 cm⁻¹ |

### STADE D : 1-benzyloxy 4-diméthylamino 3-phénylthio 3-butène 2-one

On introduit :
- 21,24 g du produit obtenu ci-dessus au stade c)
   et
- 12,1 ml de diméthylformamide diméthylacétal
et agite 1 heure 30 minutes à 70°C, laisse revenir à température ambiante, évapore à sec et obtient 24,8 g de produit attendu.

| Analyses: | |
|---|---|
| Spectre IR (CHCl₃) | |
| système conjugué | 1655 cm⁻¹ |
| | 1580 cm⁻¹ |
| aromatiques | 1496 cm⁻¹ |

### STADE E : 4-benzyloxy 3-oxo 2-(phénylthio) butanal

On introduit 24,6 g du produit obtenu ci-dessus, au stade d) dans 85 ml de solution constituée de 6 g de soude, 20 ml d'eau et 80 ml d'éthanol et chauffe à une température d'environ 60°C.

On laisse revenir à la température ambiante et verse sur un mélange de 150 ml de glace et 16 ml d'acide chlorhydrique pur.

On extrait par 180 ml puis 2 fois 100 ml d'acétate d'éthyle, lave la phase organique avec une solution aqueuse saturée en chlorure de sodium, sèche, filtre, concentre et obtient 22,5 g de produit attendu.

| Analyses: | |
|---|---|
| Spectre IR (CHCl₃) | |
| système conjugué | 1633 cm⁻¹ |
| | 1609 cm⁻¹ |
| aromatiques | 1492 cm⁻¹ |

### STADE F : 4-acétoxy 1-benzyloxy 3-phénylthio 3-butène 2-one

On introduit :
- 2,28 g du produit obtenu ci-dessus au stade E
- 0,65 ml de pyridine
   et
- 8 ml de toluène,
refroidit à -10°C, ajoute 0,57 ml de chlorure d'acétyle dans 2 ml de toluène et agite pendant environ 3 heures à -10°C.

On filtre, lave au toluène, lave la phase organique avec une solution aqueuse saturée en chlorure de sodium, filtre, sèche, élimine le toluène et obtient 3,3 g de produit attendu.

| Analyses : | |
|---|---|
| Spectre IR (CHCl₃) | |
| - C = O | 1783 cm⁻¹ |
| | 1702 cm⁻¹ |
| - C = C et | 1598 cm⁻¹ |
| aromatiques | 1587 cm⁻¹ |
| | 1496 cm⁻¹ |

### EXEMPLE 1 : (1-benzyl) (2-benzyloxyméthyl) (5-méthyl) (3-phénylthio) 4-pyridone (ne fait pas partie de l'invention)

### STADE A : (4-acétoxy) (2-benzyloxyméthyl) (6-éthoxy) (5-méthyl) (3-phénylthio) 4H(5,6)-pyranne

On introduit :
- 3 g du produit obtenu au stade F de la préparation de l'exemple 1
- 16,5 ml de 1-éthylpropényléther
   et
- 33 mg d'hydroquinone,
et chauffe jusqu'à une température d'environ 85°C pendant environ 24 heures.

On laisse revenir à la température ambiante, concentre et obtient 5,7 g d'une huile brune renfermant le produit attendu. Le brut est purifié par chromatographie sur colonne de silice (éluant : hexane-acétate d'éthyle 9-1).

| Analyses : | |
|---|---|
| Spectre IR (CHCl₃) | |
| - OAc | 1734 cm⁻¹ |
| - C = C | 1625 cm⁻¹ |

aromatique 1586-1497 cm⁻¹

### STADE B : (2-benzyloxyméthyl) (4-hydroxy) (6-éthoxy) (5-méthyl) (3-phénylthio) 4H(5,6)-pyranne

On introduit :
- 4,6 g du produit obtenu ci-dessus au stade a)
- 1,417 g de carbonate de sodium
   et
- 50 ml de méthanol
et agite en présence d'une quantité catalytique de méthylate de sodium à la température ambiante pendant 16 heures.

On dilue avec de l'eau, lave la fraction organique avec une solution aqueuse saturée en chlorure d'ammonium, extrait à l'éther, sèche la phase organique, filtre, concentre et obtient 3,23 g de produit attendu sous forme d'une huile brune.

| Analyses : | |
|---|---|
| Spectre IR (CHCl₃) | |
| - OH | 3540 cm⁻¹ |
| - hétérocycle et | 1627 cm⁻¹ |
| aromatique | 1602 cm⁻¹ - 1583 cm⁻¹ |
| | 1496 cm⁻¹- 1478 cm⁻¹ |

### STADE C : (2-benzyloxyméthyl) (6-éthoxy) (5-méthyl) (3-phénylthio) 4-dihydropyrone

On introduit :
- 310 mg du produit obtenu ci-dessus au stade B
- 910 mg de Pyridinium dichromate
   et
- 5 ml de diméthylformamide
et agite à la température ambiante pendant environ 3 heures.

On dilue avec de l'eau, extrait à l'éther, filtre, sèche la phase organique, filtre à nouveau, concentre et obtient 477 mg de produit attendu sous forme d'une huile jaune.

| Analyses : | |
|---|---|
| Spectre IR (CHCl₃) | |
| - C = O | 1684 cm⁻¹ |
| - hétérocycle et | 1582 cm⁻¹ |
| aromatique | 1571 cm⁻¹ |
| | 1496 cm⁻¹ |

### STADE D : (2-benzyloxyméthyl) (5-méthyl) (3-phénylthio) 4-pyrone

On introduit :
- 477 mg du produit obtenu ci-dessus au stade C
   et
- 20 ml de toluène
et agite en présence d'une quantité catalytique d'acide para-toluènesulfonique à une température de 88°C pendant 6 heures.

On laisse revenir à la température ambiante, ajoute du carbonate de calcium, agite, filtre, concentre et obtient 350 mg de produit attendu sous forme d'huile.

| Analyses : | |
|---|---|
| Spectre IR (CHCl₃) | |
| - système conjugué | 1644 cm⁻¹ |
| | 1630 cm⁻¹ |
| - aromatique | 1604 cm⁻¹ |
| | 1580 cm⁻¹ - 1498 cm⁻¹ |

### STADE E : (1-benzyl) (2-benzyloxyméthyl) (5-méthyl) (3-phénylthio) 4-pyridone

On introduit :
- 310 mg du produit obtenu ci-dessus au stade D
- 300 mg de benzylamine
   et
- 10 ml de méthanol
et agite 48 heures à température ambiante, on évapore à sec et obtient 340 mg de produit attendu.

| Analyses : | |
|---|---|
| Spectre IR (CHCl₃) | |
| - C = O | 1635 cm⁻¹ |
| - C = C et | 1586 cm⁻¹ |
| aromatique | 1512 cm⁻¹ |
| | 1498 cm⁻¹. |

### EXEMPLE 4 : 2-(hydroxyméthyl) 5-méthyl 3-phénylthio 1-[[2'-(1H-tétrazol-5-yl) (1,1'-biphényl) 4-yl] méthyl] 4-(1H)-pyridone (produit de l'invention)

### STADE A : 5-(4'-bromométhyl 1,1-biphényl-2-yl) 1-triphénylméthyl 1H-tétrazole.

A 315 g de 5-(4'-méthyl 1,1-biphényl-2-yl) 1-triphénylméthyl 1H-tétrazole décrit dans J. Org. Chem. 1991, 56, 2395 en solution dans 4725 cm³ de dichloréthane, on ajoute 117,1 g de N-bromosuccinimide puis 13,9 g de peroxyde de benzoyle et chauffe au reflux pendant 45 minutes. On refroidit à +30°C, distille partiellement le solvant sous pression réduite, puis ajoute 1470 cm³ d'éther isopropylique, évapore de nouveau partiellement le solvant, maintient l'agitation à 20°C pendant 1 heure, essore le produit cristallisé, sèche à 40°C sous pression réduite pendant 16 heures et recueille 316,7 g de produit attendu.

### STADE B : 5-(4'-azidométhyl 1,1-biphényl-2-yl) 1H-tétrazole.

On dissout à température ambiante 11,14 g de produit obtenu au stade A et 1,56 g d'azoture de sodium dans 200 cm³ de diméthylsulfoxyde, agite 3 heures et demie, verse le milieu réactionnel dans l'eau glacée, extrait à l'acétate d'éthyle, lave à l'eau salée, sèche et évapore le solvant sous pression réduite. On obtient 10,2 g de produit utilisé tel quel pour le stade suivant.

### STADE C : 5-(4'-aminométhyl 1,1-biphényl-2-yl) 1H-tétrazole.

On reprend le produit obtenu au stade A dans 1000 cm³ de méthanol, ajoute 2 g de charbon actif à 10% de palladium et hydrogène pendant 48 heures. On filtre, rince au méthanol et évapore le solvant sous pression réduite. Après chromatographie sur silice (éluant : acétone-acétate d'éthyle-eau 5-4-1), on récupère 3,2 g de produit attendu. F > 260°C.

### STADE D : 2-(benzyloxyméthyl) 5-méthyl 3-(phénylthio) 1-[[2'-(1H-tétrazol-5-yl) (1,1'-biphényl) 4-yl] méthyl] 4-(1H)-pyridinone.

On dissout à température du reflux dans 20 cm³ de méthoxy éthanol 1,14 g de (2-benzyloxyméthyl) 5-méthyl 3-phénylthio 4-pyrone déceite dans Ann. Chem. 1985, 2261-2284 et préparé comme indiqué au stade D de l'exemple 1 et 1,14 g de l'amine préparée au stade B ci-dessus. Après 24 heures de reflyx, on laisse refroidir, évapore le solvant, chromatographie le résidu sur silice (éluant : acétone-acétate d'éthyle-eau 5-4-1) et récupère 0,92 g de produit attendu.

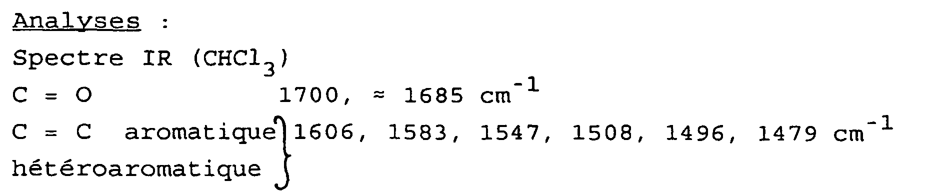

### STADE E : 2-(hydroxyméthyl) 5-méthyl 3-phénylthio 1-[[2'-(1H-tétrazol-5-yl) (1,1'-biphényl) 4-yl] méthyl] 4-(1H)-pyridone.

On chauffe 48 heures au reflux 1,43 g de produit préparé comme au stade C dans 15 cm³ d'acide acétique et 7,5 cm³ d'acide chlorhydrique concentré. On laisse revenir à température ambiante, verse sur l'eau glacée, alcalinise à pH = 14 par de la soude concentrée, extrait au chlorure de méthylène puis acidifie la phase aqueuse. On agite 1 heure à température ambiante, essore, lave à l'eau, sèche sous pression réduite à 50°C et recueille 0,5 g de produit attendu. F = 160°-163°C.

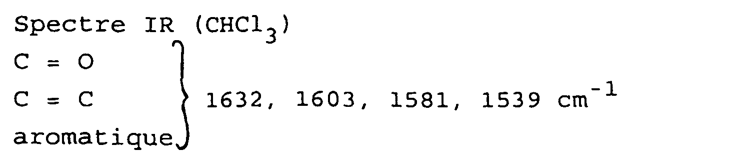

absorption complexe région OH/NH

### EXEMPLE 103 : de composition pharmaceutique

On a préparé des comprimés répondant à la formule suivante :

| | |
|---|---|
| Produit de l'exemple 1 | 10 mg |
| Excipient pour un comprimé terminé à | 100 mg |
| (détail de l'excipient : lactose, talc, amidon, stéarate de magnésium). | |

## Revendications

1. Le produit 2- (hydroxyméthyl)-5-méthyl-3-phénylthio-1- [[2' - (1H-tétrazol-5-yl) (1,1' -biphényl)-4-yl] méthyl]-4- (1H) -pyridone, ainsi que les sels d'addition avec les acides minéraux et organiques ou avec les bases minérales et organiques dudit produit.

2. A titre de médicament le produit tel que définie à la revendication 1, ainsi que les sels d'addition avec les acides minéraux et organiques ou avec les bases minérales et organiques pharmaceutiquement acceptables dudit produit.

3. Les compositions pharmaceutiques contenant à titre de principe actif, le médicament tel que défini à la revendication 2.

## Patentansprüche

1. Das Produkt 2-(Hydroxymethyl)-5-methyl-3-phenylthio-1-{[2'-(1H-tetrazol-5-yl)-(1,1'-biphenyl)-4-yl]-methyl}-4-(1H)-pyridon sowie die Additionssalze des genannten Produktes mit Mineralsäuren und organischen Säuren oder mit Mineralbasen und organischen Basen.

2. Als Arzneimittel das Produkt wie in Anspruch 1 definiert sowie die Additionssalze des genannten Produktes mit pharmazeutisch akzeptablen Mineralsäuren und organischen Säuren oder mit Mineralbasen und organischen Basen.

3. Pharmazeutische Zusammensetzungen, enthaltend als Wirkstoff das Arzneimittel wie in Anspruch 2 definiert.

## Claims

1. The product 2-(hydroxymethyl)-5-methyl-3-phenylthio-1-[[2'-(1H-tetrazol-5-yl)-1,1'-biphenyl-4-yl]methyl]-4(1H)-pyridone, and the addition salts with inorganic and organic acids or with inorganic and organic bases of the said product.

2. As medicament, the product as defined in Claim 1, and the addition salts with pharmaceutically acceptable inorganic and organic acids or with pharmaceutically acceptable inorganic and organic bases of the said product.

3. Pharmaceutical compositions comprising, as active principle, the medicament as defined in Claim 2.
